# EUROPEAN PATENT SPECIFICATION

(11) **EP 2 519 187 B1**
(45) Date of publication and mention of the grant of the patent: **30.09.2015**
(21) Application number: 11782709.7
(22) Date of filing: 28.10.2011
(51) Int. Cl.: A61F 2/966, A61F 2/97

(54) **INTRODUCER ASSEMBLY**
EINFÜHRVORRICHTUNG
ENSEMBLE D'INTRODUCTION

(30) Priority: 29.10.2010 GB 201018301
(43) Date of publication of application: 07.11.2012
(73) Proprietor: Cook Medical Technologies LLC, Bloomington, IN 47402 (US)
(72) Inventor: JENSEN, Anders, Ginge, DK-4100 Ringsted (DK)
(74) Representative: Jehan, Robert
(86) International application number: PCT/US2011/058368
(87) International publication number: WO 2012/058580

(56) References cited:
- DE-A1- 10 219 194
- US-A1- 2007 233 222
- US-A1- 2010 004 674

## Description

### Technical Field

The present invention relates to an introducer assembly for the deployment of implantable medical device such as stents, stent grafts, vena cava filters, occlusion devices and the like.

### Background Art

Medical devices such as stents, stent grafts and the like are typically introduced endoluminally into a patient by means of an elongate introducer or deployment assembly. The device is carried within the assembly in a radially compressed state during the deployment operation. Typically, the introducer includes an outer sheath which covers the entirety of the assembly including the medical device carried thereby. The medical device is typically carried on a carrier element, such as a pusher rod. Especially in the case of a self-expanding device, this is retained in a contracted state by retention mechanisms such as restraining wires, restraining caps and so on.

The introducer assembly is inserted percutaneously into the patient up to the site at which the medical device is to be deployed. Once in this position, the outer sheath is retracted so as to expose the medical device. The device is then released or otherwise expanded until it engages the vessel walls and held in place. In the case of a self-expanding device, such as a self-expanding stent, restraining wires hold the proximal and distal ends of the device so that this is retained in a restrained condition once the outer sheath has been retracted. The device is deployed by releasing the restraining mechanisms, typically by releasing the proximal end of the device first.

The various known introducer assemblies are relatively complex devices which require deployment to be carried out in a plurality of stages, for which there are provided various mechanisms operable by a clinician in a predetermined sequence to ensure correct deployment.

There have been a wide variety of deployment devices disclosed, a few examples being in US-2007/0233222, US-5,980,533, US-5,158,548 and US-7,329,275.

US 2007/0233222 discloses a device for delivering and deploying a radially expandable prosthesis. A prosthesis retention section comprises a cover that covers at least a proximal portion and a distal portion of the prosthesis. The cover comprises a distal sheath portion that covers a distal portion of the prosthesis and a proximal sheath portion that covers a proximal portion of the prosthesis. During a procedure the cover is withdrawn to expose the prosthesis, thereby allowing the prosthesis to expand into the body lumen. The prosthesis expands medially into the body lumen, that is a central portion of the prosthesis 12 expands before the end portions expand.

### Disclosure of The Invention

The present invention seeks to provide an improved introducer assembly.

According to an aspect of the present invention, there is provided an introducer assembly including an implantable medical device carried thereon; the implantable medical device being self-expanding and including a first end, a second end and a device length; the introducer assembly having a proximal and a distal end, wherein the distal end is insertable into a patient's vasculature; the introducer assembly including a device carrier element for carrying the implantable medical device, wherein the first end of the medical device is located proximate the distal end of the introducer assembly relative to the second end thereof; a first sheath element having a length at least as great as the length of the medical device and a distal end proximate the distal end of the introducer assembly; a sheath carrier coupled the distal end of the first sheath element and to the distal end of the introducer assembly; the sheath carrier being movable in a longitudinal direction relative to the device carrier; a second sheath element extending in a proximal direction of the medical device towards an external manipulation end of the assembly, the first and second sheath elements having substantially the same external diameters; the first sheath element having a contracted position covering the entirety of the medical device and an extended position, wherein as the sheath carrier is moved distally from its contracted position towards its extended position the first sheath element exposes the medical device from its second end first, thereby providing for the deployment of the medical device from its second end first.

According to another aspect of the present invention, there is provided an introducer assembly including an implantable medical device carried thereon; the implantable medical device being self-expanding and including a first end, a second end and a device length; the introducer assembly having a proximal and a distal end, wherein the distal end is insertable into a patient's vasculature; the introducer assembly including a device carrier element for carrying the implantable medical device, wherein the first end of the medical device is located proximate the distal end of the introducer assembly relative to the second end thereof; a first sheath element having a length sufficient to cover the majority of the length of the medical device and a distal end proximate the distal end of the introducer assembly; a sheath carrier coupled the distal end of the first sheath element and to the distal end of the introducer assembly; the sheath carrier being movable in a longitudinal direction relative to the device carrier; a second sheath element extending in a proximal direction of the medical device towards an external manipulation end of the assembly, the first and second sheath elements having substantially the same external diameters; the first sheath element having a contracted position covering the substantially the entirety of the medical device and an extended position, wherein as the sheath carrier is moved distally from its contracted position towards its extended position the first sheath element exposes the medical device from its second end first, thereby providing for the deployment of the medical device from its second end first.

In connection with this aspect of the present invention, the length of the sheath element is such as to be able to restrain the majority of the medical device when the only the second end of the medical device is not covered by the sheath and to provide for the medical device to deploy only as the sheath element is moved distally.

This structure enables the deployment of a medical device, such as a stent or stent graft, from its distal end first, that is in what could be termed a reverse arrangement to that of prior art introducer assemblies. Furthermore, the assembly can be made very simple, particularly compared to many known introducers. This has the advantage of being able to reduce the number of components used as well as reducing the number of steps which have to be performed by a clinician to deploy a medical device. Reducing the number of components of the device, particularly at its distal end, also enables the device to have a small outer diameter. Reducing the number of deployment steps facilitates the deployment procedure and can speed this up.

This deployment procedure also results in the medical device being deployed in the wider part of the lumen first, which is advantageous, as well as being able to position the device accurately with respect to side or branching vessels.

Moreover, being able to deploy a medical device from its distal end first makes the introducer assembly suitable also for deploying medical devices in a downstream direction, that is when flow in a vessel is going in a direction from the proximal end of the introducer assembly towards the distal end thereof. The introducer assembly can also be used in conventional manner, that is in a counter current direction.

Advantageously, the device carrier element is a cannula or catheter. This enables the carrier device to be used as a guide wire catheter for instance.

In the preferred embodiment, the sheath carrier includes a dilator tip at the distal end thereof.

The carrier device preferably includes a pusher element abutting the distal end of the medical device.

### Brief Description of the Drawings

Embodiments of the present invention are described below, with reference to the accompanying drawings, in which:
Figure 1 is a schematic cross-sectional view of a preferred embodiment of introducer assembly;
Figure 2 is a schematic diagram of the assembly of Figure 1 at the start of a stent deployment process;
Figure 3 is a view of the assembly of Figure 2 showing the stent substantially completely deployed; and
Figure 4 shows the assembly of Figure 3 once the stent has been fully deployed and in which the introducer assembly can be withdrawn from the patient's lumen.

### Description of the Preferred Embodiments

In the description which follows, when referring to the ends of an introducer assembly, the term proximal is used to denote the end of the introducer assembly closest to the clinician and which typically includes one or more handle members, valve units and so on which remain outside the patient and are manipulable by the clinician. The term distal is used to denote the other end of the introducer assembly, that is the end which is introduced into the patient's vasculature up to the deployment or treatment site.

In connection with an implantable medical device, the proximal end of the device is generally understood to refer to that end of the device closest to the heart, in an arterial application, while the distal end of the device is the end which is further downstream. In other words, the proximal end of the medical device is generally located at the distal end of the introducer assembly. For the sake of clarity, however, the description which follows and the claims refer to first and second ends of the medical device and proximal and distal ends of the introducer assembly.

The drawings show the distal end of an introducer assembly for deploying, in the example shown, a stent into the vasculature of a patient. The proximal end of the introducer assembly is not shown in the drawings or described herein in detail as it is envisaged that this is formed of conventional components or otherwise components having a form and function well known or otherwise readily envisaged by the person skilled in the art.

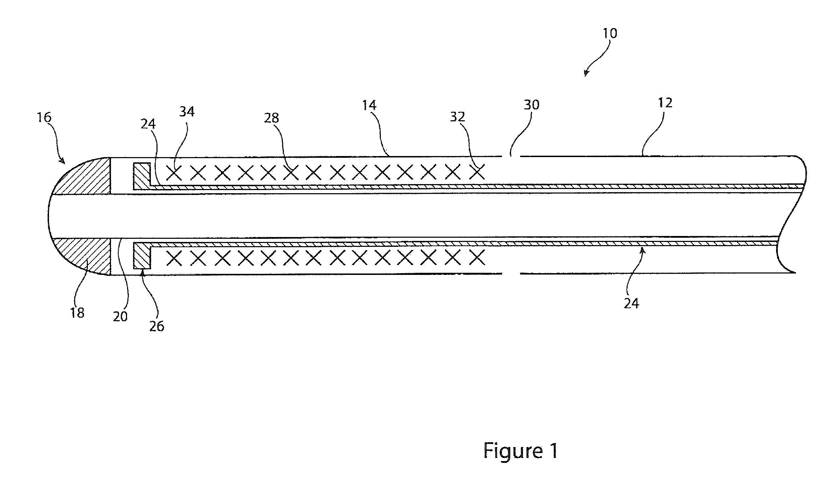

Referring to Figure 1, this is a cross-sectional view of the distal end of the preferred embodiment of introducer assembly for deploying an implantable medical device in a patient. The assembly 10 includes an outer sheath which is formed in two parts, a proximal sheath element 12 and a distal sheath element 14. The proximal sheath element 12 extends to the proximal end of the introducer assembly 12 and would typically be coupled to a handle unit which houses one or more haemostatic valves and the manipulation units used by the clinician to operate the introducer. The distal sheath element 14 extends to the distal end 16 of the introducer assembly 10 and is coupled to a sheath carrier, which in this embodiment includes a dilator tip 18.

The dilator tip 18 is typically formed of a relatively soft material and being pointed or rounded at its tip. In Figure 1 the dilator tip 18 is shown to be rounded but this may have the conventional long conical shape used in many introducer assemblies, particularly those produced by the applicants.

The assembly 10 is provided with a first catheter element 20 which forms part of the distal sheath element carrier and which extends from the proximal end of the introducer assembly to the distal end 16 and in this example all the way through the dilator tip 18, thereby to provide a guide wire lumen in the centre of the catheter 20. A guide wire (not shown), of conventional form, can be located within the guide wire lumen and thereby used for the introduction of the assembly 10 by the well known Seldinger technique.

Located concentrically around the first catheter 20 is a separate catheter or cannula 24 which will hereinafter be termed a device carrier 24. The device carrier 24 extends, in this embodiment, from the proximal (outside) end of the introducer assembly 10 to proximate the dilator tip 18. The carrier element 24 is provided with an annular shoulder 26 at its distal end. The carrier element 24 is able to slide in reciprocating manner on the guide wire catheter 20.

Carried on the carrier element 24 is an implantable medical device, in this example, a self-expanding stent 28. The stent 28 is shown in schematic form in the drawings and can be any suitable stent, such as the applicant's Zilver ™ stent or any other of the numerous stents produced by the applicants or on the market. The stent 28 is typically formed of a plurality of stent rings coupled to one another by tie bars. It can be made of any suitable self-expanding material such as a spring material but more preferably of a shape memory material such as Nitinol or other metal alloy or shape memory polymer.

As can be seen in Figure 1, when the introducer assembly 10 is in what could be described a closed state, the stent 28 is held within the entirety of the length of the distal sheath segment 14, such that the junction 30 between the proximal 12 and distal 14 segments of the sheath is beyond the extremity of the second end 32, that is the distal end, of the stent 28. Therefore, the entirety of the length of the stent 28 is held within the distal segment 14 of the sheath.

It is envisaged that a short part of the stent 28 could extend beyond the junction 30 in some embodiments but this is not preferred.

The first end 34 of the stent 28, that is its proximal end, is located typically in abutment with the annular shoulder 26. In this manner, and as described below, the shoulder 26 acts as a pusher element during the deployment of the stent 28. (In practice the shoulder will act as a pulling element to pull the stent 28 towards the proximal end of the introducer.)

The carrier catheter 24 may be provided with additional stop elements, such as another annular shoulder equivalent to the shoulder 26, to retain the second or distal end 32 of the stent in position. Such a shoulder would be located in contact with or very close to the second end 32 of the stent 38.

It is envisaged that in some embodiments there will be provided no additional constraining mechanism (in addition to the sheath 14) to maintain the stent 28 in a radially compressed condition on the introducer assembly. However, in some embodiments there may be provided additional restraining elements, such as restraining wires, restraining caps or any combination of these (all of a form known in the art). Any suitable restraining mechanism could be used.

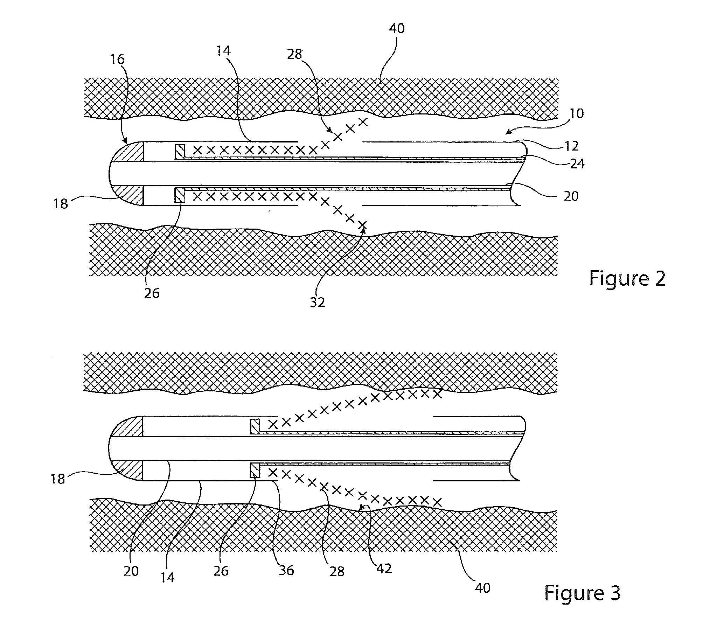

Referring now to Figure 2, this shows the distal end of the introducer assembly 10 located within a vessel 40 of a patient and in a condition in which the distal sheath section 14 has been moved distally (to the left in the drawing) of the proximal sheath segment 12 and distally relative to the carrier catheter 24 and in particular the shoulder 26. For ease of reference, it can be described with reference to Figure 2 that the distal end (formed of the distal sheath segment 14 and dilator tip 16) has been moved to the left.

As a result of this movement, the second end 32 (the distal end) of the stent becomes exposed in the widening of the gap 30 between the proximal 12 and distal 14 segments of the sheath. Due to the self-expanding characteristics of the stent 28, this begins to expand from its end 32 first, as can be seen in Figure 2.

With reference to Figure 3, further movement of the sheath section 14 in the distal direction (to the left in the drawing) causes the end 36 of the distal sheath segment 14 to move relatively closer to the stop shoulder 26, thereby exposing progressively more of the stent 28, which can thus expand radially outwardly and thereby onto and against the walls 40, 42 of the vessel 40.

It is to be understood that this deployment operation shown in Figure 3 can be achieved by continued movement of the distal sheath section 14 in a distal direction (to the left as viewed in the drawing) or by retraction in the proximal direction of the carrier catheter 24 and thus of the stop shoulder 26, so as in effect to push the stent 28 out of the introducer assembly 10. Similarly, a combination of these two movements could be carried out (that is simultaneous movement in a distal direction of the distal sheath section 14 and retraction of the carrier catheter 24).

This structure of the introducer assembly 10 causes the stent 28 to be deployed from its distal end 32 first, with its first or proximal end 34 be deployed last and once the sheath segment 14 no longer applies sufficient restraining force on the end 34. (Where additional restraining elements are provided, the first or proximal end 34 of the stent 28 could be used to keep the end 34 constrained until the appropriate time, when the restraining mechanism is released.)

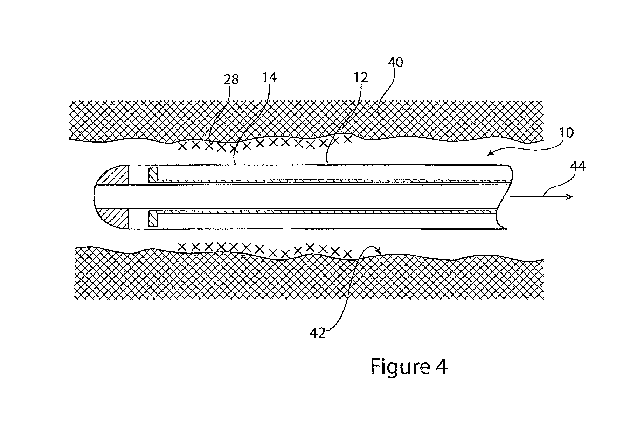

Referring now to Figure 4, the stent 28 is shown fully deployed within the lumen 40 and typically held in place against the lumen walls 42 by the expansion force produced by the stent 28. The stent 28 can also be provided with anchoring elements such as barbs.

The introducer assembly 10 is shown in Figure 4 to have been closed again, that is the distal sheath section 14 brought against the proximal sheath segment 12. The assembly 10 can be withdrawn from the patient's vessel through the deployed stent 28 in the direction of the arrow 44. Given the corresponding diameters of the sheath sections 12, 14 when closed they present a substantially smooth outer surface to avoid snagging on the device or on the lumen walls.

This structure of introducer assembly 10 provides a number of advantages. First, the assembly can be made to be very simple, that is to consist, at its distal end, of substantially only the components shown in the drawings and described above. Thus, the introducer assembly can be made less complex than known introducer assemblies and also much smaller because of the lack of other distal end deployment components typically found in other introducer assemblies.

It is also considered advantageous to be able to deploy the medical device 28 from its distal end first, as shown in Figures 2 and 3. This makes it possible to effect the deployment in a smoother manner than can be achieved with some prior art assemblies. Particularly, the structure enables an end of the medical device (of the stent 28) to be deployed first in a patient's lumen, and in a gradual manner. Some prior art systems deploy the central portion of the device first whilst its two ends remain restrained, the latter springing out from their constrained positions the moment they are released. Any sudden movement by the medical device can cause less than optimum positioning and deployment.

The structure disclosed herein can also provide for simpler and more effective repositioning of the medical device during the deployment operation, for instance by being able to move this in a distal direction as it is being deployed.

The introducer assembly disclosed herein can be used to deploy medical devices both when there is what could be termed a downstream flow (from the distal end 16 of the device falls of the proximal end) and also in an upstream direction (that, is when flow within the vessel 40 is from a proximal to distal direction with reference to the introducer assembly 10).

Although the preferred embodiment described above has the distal sheath segment 14 covering the entirety of the medical device 28, in other embodiments the second or distal end of the medical device 28 might extend a short distance beyond the end 36 of the sheath 28 and beyond the joining region 30 of the sheath elements 12, 14. The extent of protrusion beyond the sheath element 14 would be minimal and not sufficient to prevent the sheath element 14 from restraining the medical device 28 until this is moved distally and thus to provide for deployment of the medical device 28 in the manner described above in connection with the preferred embodiment.

The assembly 10 shown in the drawings and described above could be used in deploying a variety of medical devices, not just stents. It could be used for deploying, for instance, stent grafts, vena cava filters, occluders and any other medical device able to be deployed by such introducer assemblies.

## Claims

1. An introducer assembly (10) including an implantable medical device (28) carried thereon; the implantable medical device being self-expanding and including a first end (34), a second end (32) and a device length; the introducer assembly having a proximal and a distal end (16), wherein the distal end is insertable into a patient's vasculature; the introducer assembly including a device carrier element (24) for carrying the implantable medical device, wherein the first end of the medical device is located proximate the distal end of the introducer assembly relative to the second end thereof; a first sheath element (14) having a length at least as great as the length of the medical device and a distal end proximate the distal end of the introducer assembly; a sheath carrier coupled to the distal end of the first sheath element and to the distal end of the introducer assembly; the sheath carrier being movable in a longitudinal direction relative to the device carrier; a second sheath element (12) extending in a proximal direction of the medical device towards an external manipulation end of the assembly, the first and second sheath elements having substantially the same external diameters; the first sheath element having a contracted position covering the entirety of the medical device and an extended position, wherein as the sheath carrier is moved distally from its contracted position towards its extended position the first sheath element exposes the medical device from its second end first, thereby providing for the deployment of the medical device from its second end first.

2. An introducer assembly (10) according to claim 1, wherein the second end (32) of the medical device (28) is radially constrained solely by the sheath.

3. An introducer assembly (10) according to claim 1, wherein the first (34) and second (32) ends of the medical device (28) are constrained solely by the sheath.

4. An introducer assembly (10) according to claim 1, wherein the device carrier element (24) is a cannula or catheter.

5. An introducer assembly (10) according to claim 4, wherein the device carrier element (24) provides a guide lumen.

6. An introducer assembly (10) according to claim 1, wherein the sheath carrier includes a dilator tip (18) at the distal end (16) thereof.

7. An introducer assembly (10) according to claim 1, wherein the medical device (28) is a stent.

8. An introducer assembly (10) according to claim 1, including a pusher element (26) abutting the first end of the medical device.

9. An introducer assembly (10) according to claim 1, including a pusher element abutting the second end of the medical device.

10. An introducer assembly (10) including an implantable medical device (28) carried thereon; the implantable medical device being self-expanding and including a first end (34), a second end (32) and a device length; the introducer assembly having a proximal and a distal end (16), wherein the distal end is insertable into a patient's vasculature; the introducer assembly including a device carrier element (24) for carrying the implantable medical device, wherein the first end of the medical device is located proximate the distal end of the introducer assembly relative to the second end thereof; a first sheath element (14) having a length sufficient to cover the majority of the length of the medical device and a distal end proximate the distal end of the introducer assembly; a sheath carrier coupled to the distal end of the first sheath element and to the distal end of the introducer assembly; the sheath carrier being movable in a longitudinal direction relative to the device carrier; a second sheath element (12) extending in a proximal direction of the medical device towards an external manipulation end of the assembly, the first and second sheath elements having substantially the same external diameters; the first sheath element having a contracted position covering substantially the entirety of the medical device and an extended position, wherein as the sheath carrier is moved distally from its contracted position towards its extended position the first sheath element exposes the medical device from its second end first, thereby providing for the deployment of the medical device from its second end first.

## Patentansprüche

1. Einführbaugruppe (10), welche eine darauf getragene implantierbare medizinische Vorrichtung (28) aufweist; wobei die implantierbare medizinische Vorrichtung selbstexpandierend ist und ein erstes Ende (34), ein zweites Ende (32) und eine Vorrichtungslänge aufweist; wobei die Einführbaugruppe ein proximales und ein distales Ende (16) aufweist, wobei das distale Ende in das Gefäßsystem eines Patienten einführbar ist; wobei die Einführbaugruppe ein Vorrichtungsträgerelement (24) zum Tragen der implantierbaren medizinischen Vorrichtung aufweist, wobei sich das erste Ende der medizinischen Vorrichtung unmittelbar neben dem distalen Ende der Einführbaugruppe relativ zu dem zweiten Ende davon befindet; ein erstes Hüllelement (14) mit einer Länge, die mindestens so lang ist wie die Länge der medizinischen Vorrichtung, und einem distalen Ende unmittelbar neben dem distalen Ende der Einführbaugruppe; einen Hüllenträger, der an das distale Ende des ersten Hüllelementes und an das distale Ende der Einführbaugruppe gekoppelt ist; wobei der Hüllenträger in einer Längsrichtung relativ zu dem Vorrichtungsträger beweglich ist; ein zweites Hüllelement (12), das sich in einer proximalen Richtung der medizinischen Vorrichtung in Richtung eines externen Manipulationsendes der Baugruppe erstreckt, wobei das erste und zweite Hüllelement im Wesentlichen die gleichen Außendurchmesser aufweisen; wobei das erste Hüllelement eine zusammengezogene Position, in welcher die Gesamtheit der medizinischen Vorrichtung bedeckt ist, und eine ausgefahrene Position aufweist, wobei, wenn der Hüllenträger distal von seiner zusammengezogenen Position in Richtung seiner ausgefahrenen Position bewegt wird, das erste Hüllelement die medizinische Vorrichtung mit ihrem zweiten Ende zuerst freilegt, wodurch für den Einsatz der medizinischen Vorrichtung mit ihrem zweiten Ende zuerst gesorgt wird.

2. Einführbaugruppe (10) nach Anspruch 1, wobei das zweite Ende (32) der medizinischen Vorrichtung (28) radial lediglich durch die Hülle eingeschränkt ist.

3. Einführbaugruppe (10) nach Anspruch 1, wobei das erste (34) und zweite (32) Ende der medizinischen Vorrichtung (28) lediglich durch die Hülle eingeschränkt sind.

4. Einführbaugruppe (10) nach Anspruch 1, wobei das Vorrichtungsträgerelement (24) eine Kanüle oder ein Katheter ist.

5. Einführbaugruppe (10) nach Anspruch 4, wobei das Vorrichtungsträgerelement (24) ein Führungslumen bereitstellt.

6. Einführbaugruppe (10) nach Anspruch 1, wobei der Hüllenträger eine Dilatatorspitze (18) an dem distalen Ende (16) davon aufweist.

7. Einführbaugruppe (10) nach Anspruch 1, wobei die medizinische Vorrichtung (28) ein Stent ist.

8. Einführbaugruppe (10) nach Anspruch 1, welche ein Schieberelement (26) aufweist, das an das erste Ende der medizinischen Vorrichtung grenzt.

9. Einführbaugruppe (10) nach Anspruch 1, welche ein Schieberelement aufweist, das an das zweite Ende der medizinischen Vorrichtung grenzt.

10. Einführbaugruppe (10), welche eine darauf getragene implantierbare medizinische Vorrichtung (28) aufweist; wobei die implantierbare medizinische Vorrichtung selbstexpandierend ist und ein erstes Ende (34), ein zweites Ende (32) und eine Vorrichtungslänge aufweist; wobei die Einführbaugruppe ein proximales und ein distales Ende (16) aufweist, wobei das distale Ende in das Gefäßsystem eines Patienten einführbar ist; wobei die Einführbaugruppe ein Vorrichtungsträgerelement (24) zum Tragen der implantierbaren medizinischen Vorrichtung aufweist, wobei sich das erste Ende der medizinischen Vorrichtung unmittelbar neben dem distalen Ende der Einführbaugruppe relativ zu dem zweiten Ende davon befindet; ein erstes Hüllelement (14) mit einer Länge, die ausreichend ist, um den Großteil der Länge der medizinischen Vorrichtung zu bedecken, und einem distalen Ende unmittelbar neben dem distalen Ende der Einführbaugruppe; einen Hüllenträger, der an das distale Ende des ersten Hüllelementes und an das distale Ende der Einführbaugruppe gekoppelt ist; wobei der Hüllenträger in einer Längsrichtung relativ zu dem Vorrichtungsträger beweglich ist; ein zweites Hüllelement (12), das sich in einer proximalen Richtung der medizinischen Vorrichtung in Richtung eines externen Manipulationsendes der Baugruppe erstreckt, wobei das erste und zweite Hüllelement im Wesentlichen die gleichen Außendurchmesser aufweisen; wobei das erste Hüllelement eine zusammengezogene Position, in welcher im Wesentlichen die Gesamtheit der medizinischen Vorrichtung bedeckt ist, und eine ausgefahrene Position aufweist, wobei, wenn der Hüllenträger distal von seiner zusammengezogenen Position in Richtung seiner ausgefahrenen Position bewegt wird, das erste Hüllelement die medizinische Vorrichtung mit ihrem zweiten Ende zuerst freilegt, wodurch für den Einsatz der medizinischen Vorrichtung mit ihrem zweiten Ende zuerst gesorgt wird.

## Revendications

1. Ensemble d'introduction (10) comprenant un dispositif médical implantable (28) supporté sur celui-ci ; le dispositif médical implantable étant auto-expansible et comprenant une première extrémité (34), une seconde extrémité (32) et une longueur de dispositif ; l'ensemble d'introduction comportant une extrémité proximale et une extrémité distale (16), l'extrémité distale pouvant être insérée dans le système vasculaire d'un patient ; l'ensemble d'introduction comprenant un élément de support de dispositif (24) destiné à supporter le dispositif médical implantable, la première extrémité du dispositif médical étant située à proximité de l'extrémité distale de l'ensemble d'introduction par rapport à sa seconde extrémité ; un premier élément formant gaine (14) présentant une longueur au moins égale à la longueur du dispositif médical et une extrémité distale à proximité de l'extrémité distale de l'ensemble d'introduction ; un support de gaine accouplé à l'extrémité distale du premier élément formant gaine et à l'extrémité distale de l'ensemble d'introduction ; le support de gaine étant mobile dans une direction longitudinale par rapport au support de dispositif ; un second élément formant gaine (12) s'étendant dans une direction proximale du dispositif médical vers une extrémité de manipulation externe de l'ensemble, les premier et second éléments formant gaines présentant essentiellement le même diamètre externe ; le premier élément formant gaine ayant une position contractée recouvrant la totalité du dispositif médical et une position étendue, le premier élément formant gaine exposant le dispositif médical en commençant par sa seconde extrémité, à mesure que le support de gaine est déplacé dans le sens distal de sa position contractée vers sa position étendue, ce qui permet le déploiement du dispositif médical en commençant par sa seconde extrémité.

2. Ensemble d'introduction (10) selon la revendication 1, dans lequel la seconde extrémité (32) du dispositif médical (28) est contenue radialement uniquement par la gaine.

3. Ensemble d'introduction (10) selon la revendication 1, dans lequel les première (34) et seconde (32) extrémités du dispositif médical (28) sont contenues uniquement par la gaine.

4. Ensemble d'introduction (10) selon la revendication 1, dans lequel l'élément de support de dispositif (24) est une canule ou un cathéter.

5. Ensemble d'introduction (10) selon la revendication 4, dans lequel l'élément de support de dispositif (24) forme une lumière de guidage.

6. Ensemble d'introduction (10) selon la revendication 1, dans lequel le support de gaine comprend un embout de dilatation (18) au niveau de son extrémité distale (16).

7. Ensemble d'introduction (10) selon la revendication 1, dans lequel le dispositif médical (28) est un stent.

8. Ensemble d'introduction (10) selon la revendication 1, comprenant un élément formant poussoir (26) en appui contre la première extrémité du dispositif médical.

9. Ensemble d'introduction (10) selon la revendication 1, comprenant un élément formant poussoir en appui contre la seconde extrémité du dispositif médical.

10. Ensemble d'introduction (10) comprenant un dispositif médical implantable (28) supporté sur celui-ci ; le dispositif médical implantable étant auto-expansible et comprenant une première extrémité (34), une seconde extrémité (32) et une longueur de dispositif ; l'ensemble d'introduction comportant une extrémité proximale et une extrémité distale (16), l'extrémité distale pouvant être insérée dans le système vasculaire d'un patient ; l'ensemble d'introduction comprenant un élément de support de dispositif (24) destiné à supporter le dispositif médical implantable, la première extrémité du dispositif médical étant située à proximité de l'extrémité distale de l'ensemble d'introduction par rapport à sa seconde extrémité ; un premier élément formant gaine (14) présentant une longueur suffisante pour recouvrir la majeure partie de la longueur du dispositif médical et une extrémité distale à proximité de l'extrémité distale de l'ensemble d'introduction ; un support de gaine accouplé à l'extrémité distale du premier élément formant gaine et à l'extrémité distale de l'ensemble d'introduction; le support de gaine étant mobile dans une direction longitudinale par rapport au support de dispositif ; un second élément formant gaine (12) s'étendant dans une direction proximale du dispositif médical vers une extrémité de manipulation externe de l'ensemble, les premier et second éléments formant gaines présentant essentiellement le même diamètre externe ; le premier élément formant gaine ayant une position contractée recouvrant essentiellement la totalité du dispositif médical et une position étendue, le premier élément formant gaine exposant le dispositif médical en commençant par sa seconde extrémité, à mesure que le support de gaine est déplacé dans le sens distal de sa position contractée vers sa position étendue, ce qui permet le déploiement du dispositif médical en commençant par sa seconde extrémité.
